(19) **Europäisches Patentamt**
**European Patent Office**
**Office européen des brevets**

(11) **EP 2 301 561 A1**

(12) **EUROPEAN PATENT APPLICATION**
published in accordance with Art. 153(4) EPC

(43) Date of publication:
**30.03.2011 Bulletin 2011/13**

(21) Application number: **09746670.0**

(22) Date of filing: **15.05.2009**

(51) Int Cl.:
*A61K 38/00* (2006.01)    *A61P 27/02* (2006.01)
*C12N 15/09* (2006.01)    *C12P 21/02* (2006.01)

(86) International application number:
**PCT/JP2009/059052**

(87) International publication number:
**WO 2009/139464 (19.11.2009 Gazette 2009/47)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **15.05.2008 JP 2008128418**

(71) Applicant: **R-Tech Ueno, Ltd.**
**Tokyo 100-0011 (JP)**

(72) Inventors:
• **MASHIMA, Yukihiko**
**Tokyo 100-0011 (JP)**

• **KISHIMOTO, Nakayuki**
**Tokyo 100-0011 (JP)**
• **TABUCHI, Reiko**
**Tokyo 100-0011 (JP)**
• **SHIMA, Naoko**
**Tokyo 100-0011 (JP)**

(74) Representative: **Albrecht, Thomas**
**Kraus & Weisert**
**Patent- und Rechtsanwälte**
**Thomas-Wimmer-Ring 15**
**80539 München (DE)**

(54) **PHARMACEUTICAL COMPOSITION FOR TREATMENT OF DRY EYE AND/OR CORNEAL/ CONJUNCTIVAL DISORDERS**

(57) The present invention provides a pharmaceutical composition for the treatment of dry eye and/or corneal and conjunctival lesion which comprises a recombinant human serum albumin produced by a recombinant yeast obtained by transforming a yeast with a gene for human serum albumin. Further, the present invention provides a method for the treatment of dry eye and/or corneal and conjunctival lesion, which comprises administering an effective amount of a recombinant human serum albumin produced by a recombinant yeast obtained by transforming a yeast with a gene for human serum albumin to a subject in need of the treatment of eye and/or corneal and conjunctival lesion.

**EP 2 301 561 A1**

**Description**

Technical Field

The present invention provides a pharmaceutical composition for ophthalmic use comprising recombinant human serum albumin produced by recombinant yeast cells that are obtained by gene manipulation of host yeast cells. The pharmaceutical composition of the present invention is useful for the treatment of dry eye and corneal and conjunctival lesion. The present invention further provides methods for the treatment of corneal and conjunctival lesion as well as the treatment of dry eye condition using the composition of the present invention.

Background Art

Corneal and conjunctival lesion is triggered by defects of the surface to epithelium of the cornea. The cause may include pathogenic factors such as dry eye, various keratoconjunctivitis, allergy and infection of microorganisms (e.g. virus, bacteria, fungus, etc.), chemical factors such as cytotoxicity by chemicals and corrosion due to acid and alkaline, physical factors such as dryness of the surface of the eyeball, injury due to foreign matter (e.g. contact lens, etc.) and hot water, and the like. It has recently been reported that antiseptics contained in an ophthalmic composition (e.g. benzalkonium chloride, chlorobutanol, etc.) and ophthalmic agents (e.g. aminoglycoside antibiotics, non-steroidal anti-inflammatory drugs, IDU, pimaricin, etc.) cause a lesion of corneal epithelium. For the present, in order to treat the corneal and conjunctival lesion, chondroitin sulfate, glutathione, hyaluronic acid, fibronectin, EGF, and the like are administered or an artificial tear solution is also administered for the purpose of replenishing a tear solution, but the effect of these treatments are not yet sufficient.

Dry eye has been known as disorder of the tear film due to tear deficiency or excessive evaporation which causes damage to the interpalpebral ocular surface and is associated with symptoms of ocular discomfort. Recently, the Definition and Classification Subcommittee of the International Dry Eye Workshop provided a new definition of dry eye as follows: dry eye is a multifactorial disease of the tears and ocular surface that results in symptoms of discomfort, visual disturbance, and tear film instability with potential damage to the ocular surface. It is accompanied by increased osmolarity of the tear film and inflammation of ocular surface. Dry eye can be classified into two major groups: aqueous tear-deficient dry eye and evaporative dry eye. Aqueous tear-deficient dry eye is due to a failure of lacrimal tear secretion. Evaporative dry eye is due to excessive water loss from the exposed ocular surface in the presence of normal lacrimal secretory function. It causes have been described as intrinsic where they are due to intrinsic disease affecting lid structures or dynamics or extrinsic where ocular surface disease occurs due to some extrinsic exposure (Non patent literature 1). There are various factors that cause dry eye. No suitable method to recover the decreased amount of tear normal has been found yet. At present, in order to treat dry eye, an artificial tear solution for the purpose of replenishing tear, and chondroitin sulfate, glutathione, hyaluronic acid, fibronectin, serum eye drops, and the like for the purpose of relieving subjective symptoms are administered, but the effects are not yet sufficient.

Human serum albumin (hereinafter, referred as HSA) is a major component of human blood plasma proteins. This protein is generated in liver and plays an important role in keeping the blood osmolality within the normal range. In addition, the protein functions as a carrier for various serum molecules. HSA is now used for the treatment of conditions associated with significant loss of body fluid such as conditions after received surgical operation, shock, burn and hypoproteinemia. For example, in a patient suffered from shock or burn, HSA is administered repeatedly to recover the decreased blood volume and improve the symptoms associated with the injury. HSA is also used for the treatment of hypoproteinemia and fetal erythroblastosis.

At present, HSA is manufactured by fractionating donated human blood. Disadvantages of thus manufactured product are high cost and difficulty in obtaining donated human blood stably. Further, there is a possibility that the donated human blood is contaminated with an unfavorable matter such as hepatitis virus. Accordingly, development of a material which can be a substitute of HSA is desired.

After the establishment of the recombinant DNA technology, various useful polypeptides have been manufactured by recombinant microorganisms. Large scale production of recombinant HSA had been achieved by employing the gene manipulation techniques and highly purified HSA product has been provided. Patent Literature 1 discloses recombinant human serum albumin (hereinafter, referred as "rHSA") generated by recombinant yeast obtained by gene manipulation of Pichia yeast. Thus obtained rHSA is now received marketing authorization from the Ministry of Health, Labour and Welfare, Japan and available on the market under the product name of "Medway® injection" from Mitsubishi Tanabe Pharma Corporation (Osaka, Japan). Similar to HSA, Medway® injection has been revealed to be useful for the treatment of conditions associated with significant loss of body fluid from the blood vessel including conditions after surgical operation, shock, heat burn and hypoproteinmia. Further, a FDA-approved recombinant human albumin Albagen™ is also available on the market from New Century Pharmaceuticals, Inc. (AL, US) as an additive for medicaments. Albagen™ is also manufactured using recombinant Pichia yeast. Non Patent Literature 2 discloses recombinant human serum albumin obtained by gene manipulation of Saccharonyces yeast. Said rHSA is also available on the market with FDA-approval under the name of Recombumin® from Novozymes Inc. as a stabilizer for medical products such as vaccines.

Patent Literature 2 discloses that human serum albumin is useful for the treatment of several ocular diseases including dry eye and corneal and conjunctival lesion. Patent Literature 3 discloses ophthalmic composition for the treatment of dry eye that comprises HSA in a concentration as low as 0.001-0.3mg/ml. According to patent literature 3, ophthalmic formulation comprising 1.0mg/ml (0.1w/v%) or more of HSA is stimulus to the eyes and causes problems such as local irritation when administered topically to the eyes.

Patent Literatures 2 and 3 disclose that HSA in general can be replaced with recombinant human albumin obtained by a conventional genetic engineering procedures. However, there is no information about the effect of recombinant human serum albumin that is obtained by gene manipulation of a yeast as the host organism on dry eye or corneal and conjunctival lesion.

Patent Literature 1: JP-A-6-100592
Patent Literature 2: WO97/039769
Patent Literature 3: JP-A-2000-319194
Non Patent Literature 1: The Ocular Surface 2007 Apr, VOL. 5, No. 2: 12-28
Non Patent Literature 2: Journal of Clinical Pharmacology 2005;45:57-67
The above documents are herein incorporated by reference.

Disclosure of Invention

Problem(s) to be solved by the invention

An object of the present invention is to provide a novel pharmaceutical composition for the treatment of dry eye and/or corneal and conjunctival lesion.

Means for solving the Problem(s)

The inventors have found that the effects of recombinant human serum albumin obtained by gene manipulation of yeast cells on dry eye and corneal and conjunctival lesion are different from the effects of human serum albumin obtained by fractionating donated human blood disclosed in the prior art references and that a lower concentration, as low as 0.001-0.3mg/ml of the recombinant human albumin is not effective for the treatment of dry eye or corneal and conjunctival lesion while a higher concentration of the recombinant human serum albumin, as high as 10mg/ml (1.0 w/v%) or more is effective for the treatment of dry eye as well as corneal and conjunctival lesion without causing eye irritancy, and completed the present invention.

The instant application provides following inventions:

(1) A pharmaceutical composition for the treatment of dry eye, which comprises recombinant human serum albumin produced by a recombinant yeast obtained by transforming a yeast with a gene for human serum albumin.
(2) A pharmaceutical composition for the treatment of corneal and conjunctival lesion, which comprises recombinant human serum albumin produced by a recombinant yeast obtained by transforming an yeast with a gene for human serum albumin.
(3) The composition according to (1) or (2), wherein the recombinant yeast is obtained by transforming an yeast of genus Pichia or genus *Saccharomyces.*
(4) The composition according to (3), wherein the yeast is *Pichia pastors* or *Saccharomyces cerevisiae.*
(5) The composition according to (1) or (2), wherein the recombinant human serum albumin has the following properties:

the purity of the recombinant human serum albumin based on the total amount of the recombinant human serum albumin and contaminants originated from the recombinant yeast cells consisting of proteins having an antigenicity different from the recombinant human serum albumin and polysaccharides is more than 99.999%, and the total amount of the contaminants originated from the recombinant yeast cells consisting of proteins having an antigenicity different from the recombinant human serum albumin and polysaccharides is less than the detection limit of enzyme immunoassay(EIA).

(6) The composition according to (1) or (2), which is for topical ocular instillation.

(7) The composition according to (1), wherein dry eye is aqueous tear-deficient dry eye or evaporative dry eye.

(8) The composition according to (1), wherein dry eye is selected from the group consisting of alacrima, xerophthalmia, Sjogren syndrome, dry keratoconjunctivitis, Stevens-Johnson syndrome, ocular pemphigoid, dry eye after ophthalmic operation, dry eye accompanied with allergic conjunctivitis, dry eye like conditions including tear decrement of VDT (Visual Display Terminal) worker and tear decrement without any systemic symptom caused by dry room due to air conditioning.

(9) The composition according to (1), wherein dry eye is evaporative dry eye.

(10) The composition according to (2), wherein the corneal and conjunctival lesion is a defect of corneal and conjunctival epithelium, corneal and conjunctival erosion or corneal and conjunctival ulcer that is caused by dry eye, keratoconjunctivitis, allergy and infection of microorganisms including virus, bacteria, and fungus, cytotoxicity by chemicals, Chemical factors, xerophthalmia, injury due to antiseptics contained in an ophthalmic composition, an active ingredient contained in an ophthalmic composition, ophthalmic operation and ophthalmic injection.

(11) Use of a recombinant human serum albumin produced by a recombinant yeast obtained by transforming a yeast with a gene for human serum albumin, for the manufacture of a pharmaceutical composition for the treatment of dry eye.

(12) Use of a recombinant human serum albumin produced by a recombinant yeast obtained by transforming a yeast with a gene for human serum albumin, for the manufacture of a pharmaceutical composition for the treatment of corneal and conjunctival lesion.

(13) A method for the treatment of dry eye, which comprises administering an effective amount of a recombinant human serum albumin produced by a recombinant yeast obtained by transforming a yeast with a gene for human serum albumin to a patient in need thereof.

(14) A method for the treatment of corneal and conjunctival lesion, which comprises administering an effective amount of recombinant human serum albumin produced by a recombinant yeast obtained by transforming a yeast with a gene for human serum albumin to a patient in need thereof.

Effect of the Invention

According to the instant application, it has been revealed that the effect of recombinant human serum albumin produced by genetically modified yeast on dry eye as well as on corneal and conjunctival lesion are different from those of human serum albumin obtained as disclosed in Patent Literature 3, i.e. by fractioning human blood. The ophthalmic composition of the present invention is useful for the treatment of dry eye and/or corneal and conjunctival lesion effectively.

Best Mode For Carrying out the Invention

In the present invention, the rHSA-producing recombinant yeast obtained by gene manipulation is not particularly restricted so long as it has been prepared by gene manipulation using a yeast as the host microorganism. Namely, either those which have been reported in literatures or those which will be developed in future may appropriately be employed. Examples of yeasts may include those belonging to the genus *Kluyveromyces, Saccharomyces* and Pichia, and in partucular, *Kluyveromyces laactis, Saccharomyces cerevisiae* and *Pichia pastoris* are preferably used. It is still preferable to use *Kluyveromyces laactis* CBS2360 strain, *Saccharomyces cerevisiae* AH22 strain (a, his 4, leu 2, can 1) and *Pichia pastoris* GTS115 strain (his 4) as the host yeast. In the present invention, perticalarly, *Pichia yeast* and *Saccharomyces* yeast, and in particular *Pichia pastoris* and *Saccharomyces cerevisiae* are preferably used.

The preparation of rHSA-producing recombinant cells, the production of rHSA by culturing the recombinant cells, and the isolation and recovery of the produced rHSA from the culture may be carried out in accordance with known methods which may be modified slightly. For example, preparation of an rHSA-producing cell strain may be effected using a process in which a natural HSA gene is used (JP-A-58-56684 (EP-A-73646), JP-A-58-90515 (EP-A-79739) and JP-A-58-150517 (EP-A-91527)), a process in which a novel human serum albumin gene is used (JP-A-62-29985 and JP-A-1-98486 (EP-A-206733)), a process in which a synthetic signal sequence is used (JP-A-1-240191 (EP-A-329127)), a process in which a serum albumin signal sequence is used (JP-A-2-167095 (EP-A-319641)), a process in which a recombinant plasmid is introduced into a chromosome (JP-A-3-72889 (EP-A-399455)), a process in which hosts are fused (JP-A-3-53877 (EP-A-409156)), a process in which a mutation is generated in a methanol containing medium, a process in which a mutant AOX2 promoter is used (JP-A-3-63598, JP-A-3-63599), a process in which HSA is expressed in B. subtilis (JP-A-62-25133 (EP-A-229712)), a process in which HSA is expressed in yeast (JP-A-60-41487 (EP-A-123544), JP-A-63-39576(EP-A-248657) and JP-A-63-74493 (EP-A-251744) and a process in which HSA is expressed in Pichia yeast (JP-A-2-104290 (EP-A-3444S9)).

Of these methods, the method in which mutation of the yeast is induced in a methanol-containing medium is carried out in the following manner. A transformant of an appropriate host, preferably a Pichia yeast, illustratively a strain GTS115 (NRRL deposition No. Y-15851), is obtained in the usual manner by introducing a plasmid, containing a transcription unit by which HSA is expressed under the control of the $AOX_1$ promoter, into the $AOX_1$ gene region of the host (cf. JP-A 2-104290). This transformant hardly grows in a medium containing methanol. In consequence, this transformant is cultured in a methanol-containing medium to generate mutation, and a strain capable of growing in the medium is isolated. Methanol concentration in the medium may range, for example, from about 0.0001 to about 5%. The medium may be either synthetic or natural. The culturing may be carried out, for example, at a temperature of approximately from 15 to 40˚C. for approximately from 1 to 1,000 hours.

Culturing of the rHSA-producing recombinant cells, i.e. producing of the rHSA may be effected by each of the methods disclosed in the above patents or by a method in which rHSA-producing recombinant yeast cells and the product are obtained in high concentrations by a fed-batch culture (a semi-batch culture). The fed-batch culture is carried out by gradually supplying a high concentration of glucose to avoid substrate inhibition against the recombinant cells (JP-A-1-219561). Culturing of the recombinant cells may also be effected by a method in which the rHSA productivity is improved by the addition of fatty acids to the culture medium (JP-A-4-293495 (EP-A-504823 and U.S. Pat. No. 5,334,512). The methods for separating and harvesting rHSA may include, for example, inactivation of protease by heat treatment (JP-A-3-103188), and suppression of coloring of the rHSA by separating rHSA from coloring components with the use of at least one member of the group of anion exchanger, hydrophobic carrier, and active charcoal (JP-A-4-54198).

A medium usually employed in the art that is supplemented with a fatty acid having from 10 to 26 carbon atoms or a salt thereof can be used as a medium for culturing the recombinant yeast, and culturing the recombinant yeast can be carried out under known conditions. The medium may be either synthetic or natural, but preferably is a liquid medium. For example, a suitable synthetic medium may be composed of: carbon sources, such as various saccharides; nitrogen sources, such as urea, ammonium salts, nitrates; trace nutrients, such as various vitamins, nucleotides; and inorganic salts, such as of Mg, Ca, Fe, Na, K, Mn, Co and Cu. An illustrative example of such a medium is YNB liquid medium, which consists of 0.7% Yeast Nitrogen Base (Difco) and 2% glucose. An illustrative example of a useful natural medium is YPD liquid medium, which consists of 1% Yeast Extract (Difco), 2% Bacto Peptone (Difco) and 2% glucose. The pH of the medium may be neutral, weakly basic or weakly acidic. In the case of a methylotrophic yeast, the medium may further be supplemented with methanol in an amount of approximately from 0.01 to 5%.

The culturing temperature preferably ranges from 15 to 43˚ C and especially 20 to 30˚ C. The culturing period ranges from about 1 to 1,000 hours. The culture may be conducted by means of static or shake culturing or culturing under agitation in batch, semi-batch or continuous culturing. Preculture in advance of the main culture is preferably conducted using a medium, for example, YNB liquid medium or YPD liquid medium. The preculture may be conducted for 10 to 100 hours at about 30˚C.

After the culture is finished, rHSA is harvested from the culture filtrate or yeast cells by a known separation method.

The purification of rHSA may be carried out by means of conventionally known procedures such as various fractionating method, absorption chromatography, affinity chromatography, gel filtration, density gradient centrifugation and dialysis. One example of the preferred purification procedure may comprise following (1) to (7) steps:

(1) ultrafiltrating the culture supernatant of human serum albumin-producing recombinant yeast cells using ultrafiltration membranes having a fractional molecular weight of from 100,000 to 500,000 and from 1,000 to 50,000;
(2) heating the fraction containing rHSA at 50˚-70˚ C. for 30 minutes to 5 hours;
(3) adjusting the pH of the fraction containing rHSA with an acid at pH 3-5;
(4) ultrafiltrating the thus treated product with an ultrafiltration membrane having a fractional molecular weight of from 100,000 to 500,000;
(5) contacting the fraction containing rHSA with a cation exchanger under the condition of pH 3-5 and a salt concentration of 0.01-0.2M; and eluting the product under the condition of pH 8-10 and a salt concentration of 0.2-0.5M;
(6) contacting the eluate with a carrier for hydrophobic chromatography under the condition of pH 6-8 and a salt concentration of 0.01-0.5M and collecting the unadsorbed fraction; and
(7) contacting the unadsorbed fraction with an anion exchanger under the condition of pH 6-8 and a salt concentration of 0.01-0.1M and collecting the unadsorbed fraction.

The above-mentioned steps may include, in place of the aforementioned step (6), or a step of contacting the eluate with a carrier for hydrophobic chromatography under the condition of pH 6-8 and salt concentration of 1-3M and then eluting the product under the condition of pH 6-8 and salt concentration of 0.01-0.5M; in place of the aforementioned step (7), a step of contacting the unadsorbed fraction with an anion exchanger under the condition of pH 6-8 and salt concentration of 0.001-0.05M and then eluting the product under the condition of pH 6-8 and salt concentration of 0.05-1M. Further, the steps may also include between the aforementioned steps (5) and (6), (6) and (7) or after (7), a step of salting out under the condition of pH 3-5 and salt concentration of 0.5-3M and collecting the precipitate fraction.

The purification procedure of rHSA may contain a decolorizing step preferably at the end of the purification procedure. The decolorizing step may be conducted by contacting the obtained crude rHSA with a chelate resin having a specific ligand. It is preferable that the carrier part of the chelate resin is one having hydrophobic properties such as a styrene/divinylbenzne copolymer and an acrylic acid/methacrylic acid copolymer. Examples of the ligand part of the chelate resin include polyol groups such as N-methylglucamine, polyamine groups (including polyalkylene polyamines such as polyethylene polyamine) having plural imino groups, amino groups, ethyleneimino groups, etc. in the molecule and thiourea groups. It is convenient to use commercially available those having a styrene/divinylbenzene copolymer carrier, such as DIAION CRB02 (ligand: N-methylglucamine group, manufactured by Mitsubishi Chemical Corporation), DIAION CR20 (ligand: - $NH(CH_2CH_2NH)nH$, manufactured by Mitsubishi Chemical Corporation) LEWATIT TP (ligand: -$NHCSNH_2$, manufactured by Bayer Corporation) and Amberlite CG4000.

Suitable conditions for this chelate resin treatment are as follows.

pH: acidic or neutral, preferably, pH 3 to 9, and more

preferably pH 4 to 7.

Period of treatment: one hour or longer, preferably 6 hours or longer.

Ionic strength: 50 mmho or lower, preferably from 1 to 10 mmho.

Mixing ratio: 0.1 to 100 g, preferably 1 to 10 g (wet basis) of resin per 250 mg of rHSA.

The degree of coloring of thus obtained rHSA, i.e. rHSA harvested from the recombinant yeast culture and purified by conducting the above steps (1)-(7), the salting out step, and conducting the chelate resin treatment, is very low. When the rHSA is formulated into a rHSA solution of 250 mg/ml (a 25% solution), the A500nm/A280nm ratio is about 0.001-0.005. The chelate resin treatment can decrease the degree of coloring to 1/2-1/10. Especially, the coloring degree at around 500nm absorbing wavelength of rHSA, i.e. red color degree is reduced to 1/3-1/10 by the chelating treatment.

The rHSA used in the present invention is a homogeneous substance having a molecular weight of about 67,000 and an isoelectric point in the range of 4.6-5.0. It consists of monomers alone and is substantially free from dimers, polymers or decomposition products. In particular, the total amount of the contaminants including dimers, polymers and decomposition products is less than about 0.01%. Further, the rHSA used in the present invention is substantially free from impurities including proteins other than rHSA and polysaccharides originating from the recombinant yeast. The purity of rHSA based on the total amount of rHSA and the impurities is higher than 99.999999%, and preferably, higher than 99.9999999%. In particular, purified rHSA that can provide a 25% rHSA solution containing equal to or less than 1ng/ml, preferably equal to or less than 0.1ng/ml of proteins other than rHSA is exemplified. Similarly, purified rHSA that can provide a 25% rHSA solution containing equal to or less than 10ng/ml, preferably equal to or less than 1ng/ml of polysaccharides is also exemplified. As used herein, "purity of rHSA" refers to the ratio of rHSA in the rHSA product that may comprise contaminant such as proteins other than rHSA and polysaccharides originating from the recombinant yeast cells that producing the rHSA. The rHSA of the present invention can provide a coloring degree determined using a 25% rHSA solution as follows: the ratio of A350/A280 is about 0.01-0.05, the ratio of A450/A280 is about 0.001-0.02 and the ratio of A500/A280 is about 0.001-0.005. The number of fatty acid molecules attached to one rHSA molecule is equal to or less than one, and preferably, equal to or less than 0.1.

According to the present invention, rHSA may be any of those obtained from a recombinant yeast that is obtained by gene manipulation including commercially available products. For example, Medway® injection (Mitsubishi Tanabe Pharma Corporation, Tokyo, Japan) can preferably be used.

As used herein, the term "corneal and conjunctival lesion" includes corneal and/or conjunctival lesion caused by pathogenic factors such as dry eye, various keratoconjunctivitis, allergy and infection of microorganisms (e.g. virus, bacteria, fungus, etc.), chemical factors such as cytotoxicity by chemicals and corrosion due to acid and alkaline, physical factors such as xerophthalmia, injury due to foreign matter (e.g. contact lens, etc.) and hot water, and the like, antiseptics contained in an ophthalmic composition (e.g. benzalkonium chloride, chlorobutanol, etc.) and ophthalmic agents (e.g. aminoglycoside antibiotics, non-steroidal anti-inflammatory drugs, IDU, pimaricin, etc.); defects of ectocornea; corneal erosion; corneal ulcer, and the like.

Further, the corneal and conjunctival lesion may comprise conditions after surgical operation such as corneal transplantation, refractive surgery including refractive keratectomy(RK), photo refractive keratectomy(PRK), laser associated in situ keratomileusis(LASIK), intro corneal ring surgery (ICRS), anterior chamber intraocular lens implantation including phakic IOL, pterygium surgery, intracapsular cataract extraction (ICCE), extracapsular cataract extraction (ECCE), posterior capsultomy, intraocular lens implantation, vitrectomy, trabeculotomy, trabeculectomy, intravitreal implantation, removal of foreign body and hordeolum excision, and injury caused by intraviteral injection and punctal occlusion.

As used herein, the term "dry eye" may include aqueous tear-deficient dry eye and evaporative dry eye. In particular, dry eye conditions such as alacrima, xerophthalmia, Sjogren syndrome, dry keratoconjunctivitis, Stevens Johnson syndrome, ocular pemphigoid and blepharitis are exemplified. Further, the term "dry eye" includes dry eye after anterior ophthalmic operation such as cataract operation and refractive surgery and that accompanied with allergic conjunctivitis, as well as dry eye like condition such as a tear decrement of VDT (Visual Display Terminal) worker and a tear decrement without any systemic symptom caused by, for example, dry room due to air conditioning.

As used herein, the term "treatment" or "treading" refers to any means of control of the conditions, including prevention, cure and relief of the conditions and arrestation or relief of development of the condition.

The pharmaceutical composition of the present invention may be in a dosage forms such as tablets, pills, powders, suspensions, capsules, suppositories, injection preparations, ointments, ophthalmic solutions(eye drops), and the like. It is particularly preferred to topically administerable ophthalmic solution or eye drops.

In case of the composition of the present invention is formulated as an ophthalmic solution, the composition may contain rHSA in an amount of about 1 to 1000 mg/ml (0.1-100w/v%), more preferably about 10 to 1000 mg/ml (1-100w/v%), and especially, about 10 to 100 mg/ml (1-10w/v%). The composition may further contain a pharmaceutically acceptable diluent.

As used herein, the "pharmaceutically acceptable diluent" may be any diluent which is used for ophthalmic composition known to persons skilled in the art, for example, water, physiological saline, artificial tear solution, and the like. The pharmaceutical composition of the present invention may further comprise various components that are generally used in ophthalmic compositions, such as stabilizers, sterilizers, buffering agents, isotonic agents, chelating agents, pH adjusters, surfactants, and the like.

When the composition is formulated as an ophthalmic solution or eye drops, the pH of the composition is preferably adjusted from 5 to 8. The ophthalmic solution may be administered in an amount of about 1 to 100 µl/eye, preferably about 10 to 50 µl/eye, and more preferably about 30-50 µl/eye for one administration.

In an another aspect, the present invention also provides a use of rHSA for the manufacture of a pharmaceutical composition of the present invention described as above.

In a further aspect, the present invention provides a method for the treatment of corneal and conjunctival lesion, which comprises administering an effective amount of rHSA to a subject in need of the treatment of corneal and conjunctival lesion. As used herein, the term "a subject in need of the treatment of corneal and conjunctival lesion" includes both of a patient who is actually suffered from corneal and conjunctival lesion and a patient suspected to be suffered from such lesion. It includes not only a patient whose corneal and conjunctical lesion has been actually recognized but also a patient who is suspected to have corneal and conjunctical lesion as well as a patient in the state where a high possibility of occurring the condition is expected, such as a patient after receiving keratoplasty.

In a still further aspect, the present invention further provides a method for the treatment of dry eye, which comprises administering an effective amount of rHSA to a subject in need of the treatment of dry eye. As used herein, the term "a subject in need of the treatment of dry eye" includes both of a patient who has the dry eye condition and a patient who is suspected to be suffered from dry eye. The administration route is not specifically limited and topical ocular administration is preferable.

In these methods of the present invention, the "effective amount" of rHSA is an amount required for the desirable treatment, and may be selected an optimum according to the patient's symptoms, age, sex, body weight, diet, other drugs used in combination and various factors which are recognized by persons skilled in the medical field. This effective amount may also vary depending on the kind or activity of rHSA, in addition to the above factors. In these methods of the present invention, the pharmaceutical composition may be administered in an amount of about 1 to 100 µl/eye, preferably about 10 to 50 µl/eye and more preferably about 30 to 50 µl/eye, about 1 to 20 times per day and more preferably, about 1 to 10 times per day, it is not intended to limit the scope of the invention.

The pharmaceutical composition used in the present invention may be co-administered with a pharmaceutically active compound other than rHSA. The term "co-administer" may include to administer the pharmaceutically active ingredient other than rHSA before, simultaneously with and after administering the pharmaceutical composition of the present invention. When the pharmaceutically active compound other than rHSA is administered simultaneously with the composition of the present invention, rHSA and the other active ingredient may be formulated together in single dosage form or respectively in separate dosage forms. For example, artificial tear solution, polysaccharide sulfate such as hyaluronic acid and chondroitin sulfate, cyclosporine, glutathione, flavin-adenine nucleotide sodium, corticosteroid, tetracycline, vitamin A (retinol) and its derivatives such as vitamin A esters including retinol palmitate and retinol acetate, and cell growth factor such as hepatocyte growth factor(HGF), epidermal growth factor(EGF), fibroblast growth factor (FGF), insulin-like growth factor(IGF), nerve growth factor (NGF), plateletderived growth factor(PDGF), transforming growth factor(TGF-a and TGF-β) and keratinocyte growth factor(KGF) may be co-administered with the pharmaceutical composition of the present invention.

The present invention will be further explained in view of test examples shown below. The test examples should not be used for limiting the scope of the instant application in any means.

Test Example 1

Rabbit Forced Eyelid Opening Dry Eye Model

Test Method

Std:JW/CSK rabbits were used. The animals were anesthetized by subcutaneous injection of urethane (2g/kg, 8mL/kg). The eyelids of the rabbits were forced to open by use of an eye speculum and the animals were held for 3 hours in the face-down position using a rabbit holder. After the forced eyelid opening was finished, the animals were sacrificed by intravenous administration of an excessive amount of 5% pentobarbital and the eyeballs were removed. $50\mu l$ of 1% methylene blue solution was dropped on the surface of the cornea of the removed eyeball and stood for one minutes. Then, the eyeball was put into a container containing 15ml of physiological saline to wash the methylene blue. The cornea was removed with a razor and soaked in acetone/sodium sulfate solution (100% acetone: 9% aqueous $Na_2SO_4$=7:3) for one night so that methylene blue was extracted from the cornea into the solution. The amount of the methylene blue in the extract solution was determined by measuring optical density at 660nm using Multskan® Spectrum Thermo. Average absorbance $\pm$standard error are shown in the Table below. The corneal injury was evaluated by the average absorbance (i.e. methylene blue absorbance). The corneal injury inhibiting ratio of each group was evaluated according to the criteria as follows:

Average methylene blue absorbance of the untreated group (Group I)=100% inhibition of corneal injury
Average methylene blue absorbance of the group received the forced eyelid opening and administered with physiological saline (Group II)= 0% inhibition of corneal injury.

The corneal injury inhibiting ratio of the test group was calculated according to the following formula:

$$\text{Corneal injury inhibiting ratio } (\%) = \frac{(\text{Group II}) - (\text{Test Group})}{(\text{Group II}) - (\text{Group I})} \times 100$$

In the above formula, (Group I), (Group II) and (Test Group) represent the average methylene blue absorbance of the respective groups. (Test Group) represents any of Groups III-VI.

Administration of the test compounds

Immediately after starting the forced eyelid opening, the animals were administered with $50\mu l$ of physiological saline, 1, 3, or 5 w/v% of rHSA solution, or 0.3% sodium hyaluronate (Hyalein® ophthalmic solution 0.3%, Santen Pharmaceutical Co., Ltd, Osaka, Japan) topically to the both eyes.
As 1, 3 and 5% rHSA solution, 25% Medway® injection (containing 25w/v% of rHSA, Mitsubishi Tanabe Pharma Corporation, Tokyo, Japan) was diluted with physiological saline to give the desired concentration.

Test Groups

Table 1

| Group | treatment | n |
|-------|-----------|---|
| I | 0hr forced eyelid opening+ no treatment | 5(10 eyes) |
| II | 3hr forced eyelid opening + physiological saline | 10(20 eyes) |
| III | 3hr forced eyelid opening + 1% rHSA | 10(20 eyes) |
| IV | 3hr forced eyelid opening + 3% rHSA | 10(20 eyes) |
| V | 3hr forced eyelid opening + 5% rHSA | 10(20 eyes) |
| VI | 3hr forced eyelid opening + 0.3% sodium hyaluronate | 10(20 eyes) |

Result

After 3 hours forced eyelid opening, the methylene blue absorbance which indicates the corneal injury was measured. Results are shown in the Table 2 below:

The corneal injury inhibiting ratio was calculated based on result of table 2.

Table 2

| Group | methylene blue absorbance (average ± SE) |
|---|---|
| I | 0.051±0.008 |
| II | 0.217±0.010 |
| III | 0.183±0.012[*] |
| IV | 0.175±0.011[*] |
| V | 0.152±0.013[**] |
| VI | 0.219±0.013 |
| **p,0.01, *p<0.05 vs. Group II (Dunnett's test) | |

The methylene blue absorbance at 660nm in the 1%, 3% and 5% rHSA treatment groups (Groups III-V) were significantly lower than that in the saline treatment group (Group II). This result supports the effect of 1%, 3% and 5% rHSA to protect the eyes from corneal injury caused by dryness of the surface of the eyeballs in a dose dependent manner.
The corneal injury inhibiting ratio of the 1, 3, and 5% rHSA treatment groups were increased in a dose dependent manner and were 21%, 25% and 39%, respectively.

Test Experimental 2

Rabbit Forced Eyelid Opening Dry Eye Model

Test Method

Std:JW/CSK rabbits were used. The animals were anesthetized by subcutaneous injection of urethane (2g/kg, 8mL/kg). The eyelids of the rabbits were forced to open by use of an eye speculum and the animals were held for 3 hours in the face-down position using a rabbit holder. After the forced eyelid opening was finished, the animals were sacrificed by intravenous administration of an excessive amount of pentobarbital and the eyeballs were removed. 50μl of 1% methylene blue solution was dropped on the surface of the cornea of the eyeball and stood for one minutes. Then, the eyeball was put into a container containing 15ml of physiological saline to wash the methylene blue. The cornea was removed with a razor and soaked in acetone/sodium sulfate solution (100% acetone: 9% aqueous $Na_2SO_4$=7:3) for one night so that methylene blue is extracted from the cornea into the solution. The amount of the methylene blue in the extract solution was determined by measuring optical dencity at 660nm (methylene blue absorbance) using Multskan® Spectrum Thermo. Average absorbance ± standard error are shown in the Table below. The corneal injury was evaluated based on the average methylene blue absorbance.

Administration of the test compounds

Immediately after starting the forced eyelid opening, the animals were administered topically to the eyes with 50μl of physiological saline to one eye and 50μl of vehicle, 0.0001%, 0.03% or 5% rHSA solution to the other eye.
In this test example, a vehicle comprising 0.28% D(+)-glucose, 0.33% $Na_2HPO_4$, 0.55% NaCl, 0.16% KCl, 0.0024% Sodium N-Acetyl-DL-tryptophan and 0.00135% sodium caprylate in water for injection was used. The 0.0001% and 0.03% rHSA solutions were prepared by diluting rHSA stock solution with the vehicle was used in this example. The rHSA stock solution comprising 28.7% of rHSA and 0.341% of NaCl manufactured by Mitsubishi Tanabe Pharma Corporation according to the method disclosed in JP-A-2000-319194 was used. The 5% rHSA solution was prepared by diluting 25% Medway® injection (containing 25w/v% of rHSA, Mitsubishi Tanabe Pharma Corporation) in the same manner as Test Example 1.

Test Groups

Table 3

| Test Group | test component | eye | n |
|---|---|---|---|
| vehicle | vehicle | test eye | 6 |
| | saline | control eye | |
| 0.0001% rHSA | 0.0001% rHSA | test eye | 6 |
| | saline | control eye | |
| 0.03% rHSA | 0.03% rHSA | test eye | 6 |
| | saline | control | |
| 5% rHSA | 5% rHSA | test eye | 6 |
| | Saline | control eye | |

Result

After the 3 hours forced eyelid opening, the methylene blue absorbance of the coroneal extract was measured in the same manner as Test Example 1. The results are shown in table 4 below:

Table 4 : Effect for the coreal injury

| Test Group | eye | methylene blue absorbance (average $\pm$ SE) |
|---|---|---|
| vehicle | test eye | $0.278 \pm 0.031$ |
| | control eye | $0.279 \pm 0.024$ |
| 0.0001% rHSA | test eye | $0.343 \pm 0.024$ |
| | control eye | $0.361 \pm 0.041$ |
| 0.03% rHSA | test eye | $0.314 \pm 0.044$ |
| | control eye | $0.310 \pm 0.046$ |
| 5% rHSA | test eye | $0.209 \pm 0.022^{*}$ |
| | control eye | $0.309 \pm 0.029$ |
| *p<0.01 vs. control eye (Paired Student's t-test) | | |

As shown in the above table, vehicle did not affect the methylene blue absorbance compared to saline. In addition, the 0.0001% as well as 0.03% rHSA treatment groups did not affect the methylene blue absorbance compared to saline. Accordingly, vehicle as well as rHSA in a concentrations of 0.0001% or 0.03% do not protect the eyes from corneal injury caused by the dryness of the surface of the eye bolls in rabbits.

In contrast, as shown in the test example 1, the methylene blue absorbance in the 5% rHSA treatment group was significantly increased vs. the control eye and the effect of the rHSA to protect from corneal lesion caused by the dryness of the surface of the eyeballs in rabbit was confirmed.

Test Example 3

Eye mucosal irritation caused by short term frequent administration of RHEA

Test Method

Kbl:JW/SPF rabbits were used. 50μl/eye of physiological saline, 3%, 5% or 10% rHSA solution or 10% HSA solution was administered topically to the left eye of the animal repeatedly for total 10 times at 30 minutes intervals. The right eye was not treated.

10% rHSA solution was prepared by diluting 25% Medway® injection (containing 25% of rHSA, Mitsubishi Tanabe Pharmaceutical, Inc.) with 1.03% aqueous sodium chloride solution. 3% and 5% rHSA solutions were prepared by further diluting thus obtained 10% rHSA solution with physiological saline. 10% HSA solution was prepared by diluting a commercially available HSA product containing 25% HSA, kenketsu *albumin* (albumin obtained by fractionating donated human blood) manufactured by Benesis Corporation with 1.03% aqueous sodium chloride solution.

Test Groups

Table 5

| Test Groups | times | intervals | duration |
|---|---|---|---|
| Saline | 10/day | 30 min. | 1 day |
| 3% rHSA | 10/day | 30 min. | 1 day |
| 5% rHSA | 10/day | 30 min. | 1 day |
| 10% rHSA | 10/day | 30 min. | 1 day |
| 10% HSA | 10/day | 30 min. | 1 day |

Observation

At 1 hour after the final administration and 24 hours after the first and final administration respectively, eye irritancy of the left eyes were observed and scored according to the criteria of Draize (Draize,J.H., Woodard, G. and Calvery, H.O.: Methods for the study of irritation and toxicity of substances applied topically to the skin and mucous membranes., J. Pharmacol. Exptl. Therap., 82, 377-390 (1944)) presented in the Table below.
Table 6

Scale for Scoring Ocular Lesions

1. Cornea
A. Opacity-Degree of Density (area which is most dense is taken for reading.)

| | |
|---|---|
| No opacity | 0 |
| Scattered or diffuse area-details of iris clearly visible | 1 |
| Easily discernible translucent areas, details of iris slightly obscured. | 2 |
| Opalescent areas, no details of iris visible, size of pupil barely discernible | 3 |
| Opaque, iris invisible. | 4 |

B. Area of Cornea Involved

| | |
|---|---|
| No opacity | 0 |
| One quarter (or less) but not zero | 1 |
| Greater than one quarter-less than one-half | 2 |
| Greater than one-half less than three quarters | 3 |
| Greater than three quarters up to whole area | 4 |

Score equals $A \times B \times 5$

2. Iris
A. Values

| | |
|---|---|
| Normal | 0 |
| Folds, congestion, swelling, circumcorneal hyperemia above normal (any or all of one these or combination of any thereof) Comparatively reaction to light | 1 |
| No reaction to light Hemorrhage; gross destruction (any one of these) | 2 |

Score equals $A \times 5$

(continued)

Scale for Scoring Ocular Lesions

3. Conjunctivae

A. Redness

| | | |
|---|---|---|
| Normal | | 0 |
| | Vessels definitely injected above normal | 1 |
| | More diffuse, deeper crimson red, individual vessels not easily discernible | 2 |
| | Diffuse beefy red | 3 |

B. Chemosis

| | | |
|---|---|---|
| | No swelling | 0 |
| | Any swelling above normal (includes nictitating membrane) | 1 |
| | Obvious swelling with partial eversion of the lids | 2 |
| | Swelling with lids about half closed | 3 |
| | Swelling with lids about half closed to completely closed | 4 |

C. Discharge

| | | |
|---|---|---|
| | No discharge | 0 |
| | Any amount different from nominal (does not include small amountobserved in inner canthus of normal animals) | 1 |
| | Discharge with moistening of the lids and hairs just adjacent to the lids | 2 |
| | Discharge with moistening of the lids and considerable area around the eye | 3 |

$$\text{Score equals } (A + B + C) \times 2$$

The sum of all scores obtained for the cornea, iris and conjunctivae are shown as indexes for eye irritancy.

Results are shown in table 7:

Evaluation for rabbit eye irritancy

Table 7

| | | time after administration (h) | | |
|---|---|---|---|---|
| Test Groups | animal No. | 1[1] | 24[2] | 24[1] |
| Saline | M101 | 0 | 0 | 0 |
| | M102 | 0 | 0 | 0 |
| | M103 | 0 | 0 | 0 |
| | Mean | 0.0 | 0.0 | 0.0 |
| 3% rHSA | M201 | 0 | 0 | 0 |
| | M202 | 0 | 0 | 0 |
| | M203 | 0 | 0 | 0 |
| | Mean | 0.0 | 0.0 | 0.0 |
| 5% rHSA | M301 | 0 | 0 | 0 |
| | M302 | 0 | 0 | 0 |
| | M303 | 0 | 0 | 0 |
| | Mean | 0.0 | 0.0 | 0.0 |
| 10% rHSA | M401 | 0 | 0 | 0 |
| | M402 | 0 | 0 | 0 |
| | M403 | 0 | 0 | 0 |
| | Mean | 0.0 | 0.0 | 0.0 |
| 10% HSA | M501 | 0 | 0 | 0 |
| | M502 | 2 | 2 | 2 |
| | M503 | 2 | 2 | 2 |

| (continued) | | | | |
|---|---|---|---|---|
| | time after administration (h) | | | |
| Test Groups | animal No. | 1[1] | 24[2] | 24[1] |
| | Mean | 1.3 | 1.3 | 1.3 |

1) time after the final administration
2) time after the first administration.

As shown in the above table, 3, 5 and 10% rHSA solution did not cause any eye irritancy at the all observed times. In contrast, 10% HSA solution caused redness of conjunctiva (score 1) in 2/3 of the animals at 1 and 24 hours after the final administration.

## Claims

1. A pharmaceutical composition for the treatment of dry eye, which comprises recombinant human serum albumin produced by a recombinant yeast obtained by transforming a yeast with a gene for human serum albumin.

2. A pharmaceutical composition for the treatment of corneal and conjunctival lesion, which comprises recombinant human serum albumin produced by a recombinant yeast obtained by transforming an yeast with a gene for human serum albumin.

3. The composition according to Claim 1 or 2, wherein the recombinant yeast is obtained by transforming an yeast of genus Pichia or genus *Saccharomyces.*

4. The composition according to Claim 3, wherein the yeast is *Pichia pastors* or *Saccharomyces cerevisiae.*

5. The composition according to Claim 1 or 2, wherein the recombinant human serum albumin has the following properties:

   the purity of the recombinant human serum albumin based on the total amount of the recombinant human serum albumin and contaminants originated from the recombinant yeast cells consisting of proteins having an antigenicity different from the recombinant human serum albumin and polysaccharides is more than 99.999%, and the total amount of the contaminants originated from the recombinant yeast cells consisting of proteins having an antigenicity different from the recombinant human serum albumin and polysaccharides is less than the detection limit of enzyme immunoassay(EIA).

6. The composition according to Claim 1 or 2, which is for topical ocular instillation.

7. The composition according to Claim 1, wherein dry eye is aqueous tear-deficient dry eye or evaporative dry eye.

8. The composition according to Claim 1, wherein dry eye is selected from the group consisting of alacrima, xerophthalmia, Sjogren syndrome, dry keratoconjunctivitis, Stevens-Johnson syndrome, ocular pemphigoid, dry eye after ophthalmic operation, dry eye accompanied with allergic conjunctivitis, dry eye like conditions including tear decrement of VDT (Visual Display Terminal) worker and tear decrement without any systemic symptom caused by dry room due to air conditioning.

9. The composition according to Claim 7, wherein dry eye is evaporative dry eye.

10. The composition according to Claim 2, wherein the corneal and conjunctiva lesion is a defect of corneal and conjunctival epithelium, corneal and conjunctival erosion or corneal and conjunctival ulcer that is caused by dry eye, keratoconjunctivitis, allergy and infection of microorganisms including virus, bacteria, and fungus, cytotoxicity by chemicals, Chemical factors, xerophthalmia, injury due to antiseptics contained in an ophthalmic composition, an active ingredient contained in an ophthalmic composition, ophthalmic operation and ophthalmic injection.

11. Use of a recombinant human serum albumin produced by a recombinant yeast obtained by transforming a yeast with a gene for human serum albumin, for the manufacture of a pharmaceutical composition for the treatment of dry eye.

12. Use of a recombinant human serum albumin produced by a recombinant yeast obtained by transforming a yeast with a gene for human serum albumin, for the manufacture of a pharmaceutical composition for the treatment of corneal and conjunctival lesion.

13. A method for the treatment of dry eye, which comprises administering an effective amount of a recombinant human serum albumin produced by a recombinant yeast obtained by transforming a yeast with a gene for human serum albumin to a patient in need thereof.

14. A method for the treatment of corneal and conjunctival lesion, which comprises administering an effective amount of recombinant human serum albumin produced by a recombinant yeast obtained by transforming a yeast with a gene for human serum albumin to a patient in need thereof.

| **INTERNATIONAL SEARCH REPORT** | International application No. |
|---|---|
| | PCT/JP2009/059052 |

A.  CLASSIFICATION OF SUBJECT MATTER
*A61K38/00*(2006.01)i, *A61P27/02*(2006.01)i, *C12N15/09*(2006.01)i, *C12P21/02* (2006.01)i

According to International Patent Classification (IPC) or to both national classification and IPC

B.   FIELDS SEARCHED

Minimum documentation searched (classification system followed by classification symbols)
A61K38/00, A61P27/02, C12N15/09, C12P21/02

Documentation searched other than minimum documentation to the extent that such documents are included in the fields searched
Jitsuyo Shinan Koho          1922-1996   Jitsuyo Shinan Toroku Koho   1996-2009
Kokai Jitsuyo Shinan Koho    1971-2009   Toroku Jitsuyo Shinan Koho   1994-2009

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X<br>Y | WO 97/39769 A1  (R-TECH UENO, LTD.),<br>30 October, 1997 (30.10.97),<br>Claims 1 to 8, 25 to 32; page 3, lines 16 to 25;<br>page 4<br>& JP 3058920 B        & US 6043213 A<br>& US 6159930 A        & EP 834320 A1 | 1,2,6-12<br>3-5 |
| X<br>Y | US 2002/0187927 A1  (TSUBOTA Kazuo et al.),<br>12 December, 2002 (12.12.02),<br>Claims 1 to 3, 5; Par. No. [0007], [0009],<br>[0024]<br>& EP 1254665 A1        & JP 2003-192612 A | 1,2,6-12<br>3-5 |
| Y | US 5986062 A  (Welfide Corp.),<br>16 November, 1999 (16.11.99),<br>& EP 1099708 A1        & JP 6-100592 A | 3-5 |

☐ Further documents are listed in the continuation of Box C.        ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered   to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>11 June, 2009 (11.06.09) | Date of mailing of the international search report<br>23 June, 2009 (23.06.09) |
|---|---|
| Name and mailing address of the ISA/<br>Japanese Patent Office | Authorized officer |
| Facsimile No. | Telephone No. |

Form PCT/ISA/210 (second sheet) (April 2007)

<table>
<tr><td>**INTERNATIONAL SEARCH REPORT**</td><td>International application No.<br>PCT/JP2009/059052</td></tr>
</table>

**Box No. II    Observations where certain claims were found unsearchable (Continuation of item 2 of first sheet)**

This international search report has not been established in respect of certain claims under Article 17(2)(a) for the following reasons:

1. ☒ Claims Nos.: 13, 14
   because they relate to subject matter not required to be searched by this Authority, namely:
   Claims 13 and 14 pertain to methods for treatment of the human body by therapy and thus relate to a subject matter which this International Searching Authority is not required, under the provisions of Rule 39.1(iv) of the Regulations under the PCT, to search.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**      ☐ The additional search fees were accompanied by the applicant's protest and, where applicable,
the                             payment of a protest fee.

                           ☐ The additional search fees were accompanied by the applicant's protest but the applicable protest
                             fee was not paid within the time limit specified in the invitation.

                           ☐ No protest accompanied the payment of additional search fees.

Form PCT/ISA/210 (continuation of first sheet (2)) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6100592 A
- WO 97039769 A
- JP 2000319194 A
- JP 58056684 A
- EP 73646 A
- JP 58090515 A
- EP 79739 A
- JP 58150517 A
- EP 91527 A
- JP 62029985 A
- JP 1098486 A
- EP 206733 A
- JP 1240191 A
- EP 329127 A
- JP 2167095 A
- EP 319641 A
- JP 3072889 A
- EP 399455 A
- JP 3053877 A
- EP 409156 A
- JP 3063598 A
- JP 3063599 A
- JP 62025133 A
- EP 229712 A
- JP 60041487 A
- EP 123544 A
- JP 63039576 A
- EP 248657 A
- JP 63074493 A
- EP 251744 A
- JP 2104290 A
- EP 3444S9 A
- JP 1219561 A
- JP 4293495 A
- EP 504823 A
- US 5334512 A
- JP 3103188 A
- JP 4054198 A

**Non-patent literature cited in the description**

- *The Ocular Surface,* April 2007, vol. 5 (2), 12-28
- *Journal of Clinical Pharmacology,* 2005, vol. 45, 57-67
- *J. Pharmacol. Exptl. Therap.,* 1944, vol. 82, 377-390